# EUROPEAN PATENT SPECIFICATION

(11) **EP 3 670 404 B1**
(45) Date of publication and mention of the grant of the patent: **12.07.2023**
(21) Application number: 18306768.5
(22) Date of filing: 20.12.2018
(51) Int. Cl.: B65G 54/02, A61L 2/18, A61L 2/20, A61L 2/22, B67B 3/00, A61L 2/06

(54) **STERILIZATION APPARATUS HAVING A CONVEYING APPARATUS**
STERILISATIONSVORRICHTUNG MIT EINER FÖRDERVORRICHTUNG
APPAREIL DE STÉRILISATION DOTÉ D'UN APPAREIL DE TRANSPORT

(43) Date of publication of application: 24.06.2020
(73) Proprietor: Sidel Participations, 76930 Octeville-sur-Mer (FR)
(72) Inventor: BORGESE, Rossana, 43126 Parma (IT); CENCI, Mattia, 43126 Parma (IT); PAMBIANCHI, Matteo, 43126 Parma (IT)
(74) Representative: Sidel Group

(56) References cited:
- EP-A1- 1 369 379
- EP-A1- 2 500 296
- EP-A1- 3 192 531
- WO-A1-2015/128113
- DE-A1-102012 107 961
- JP-A- H04 242 525

## Description

### TECHNICAL FIELD

The present invention relates to a sterilization apparatus for receptacle closures, in particular receptacle closures for receptacles being filled with a pourable product.

### BACKGROUND ART

The sterilization of packaging material, in particular within the food packaging sector, is of fundamental interest for guaranteeing the needed shelf life of the packaged products and, accordingly, the safety of the consumers. This is even more important when food products are packaged under aseptic conditions.

It is known in the art to fill any type of pourable product, in particular any type of pourable food product such as carbonated liquids (e.g. sparkling water, soft drinks and beer), non-carbonated liquids (including still water, juices, teas, sport drinks, wine, etc) and beverages containing pulps into receptacles, such as containers, vessels, jars and bottles made of base components, like glass, plastics, aluminum, steel, and composites.

In general, the receptacles prior to being filled with the pourable product are sterilized within a receptacle sterilization apparatus and are subsequently filled with the desired pourable product within a filling apparatus.

After the filling of the receptacles, typically the respective pouring openings of the receptacles are closed by the application and fastening of respective receptacle closures.

Prior to the application of the receptacle closures, the receptacle closures must be sterilized within a respective sterilization apparatus. After sterilization the sterile receptacle closures are fed to a capping apparatus, which also receives the filled receptacles.

EP3192531A1 discloses a typical sterilization apparatus for receptacle closures comprising a conveying device having a guide track housed within an isolation chamber. The guide track is designed to receive the receptacle closures at an inlet station and to guide the receptacle closures to an outlet station from where the receptacle closures are fed to a capping apparatus.

The receptacle closures are arranged in succession to one another within and/or the guide track.

A typical conveying device also comprises an actuation unit for advancing the receptacle closures along an advancement path from the inlet station to the outlet station. The actuation unit comprises a pushing element, which is configured to insert one receptacle closure at a time into the guide track, thereby bringing into contact the newly fed receptacle closure with the succession of receptacle closures already present within the guide track. A typical succession of receptacle closures comprises between 20 to 200 receptacle closures. This leads to the exertion of a pushing force onto the succession of receptacle closures.

A typical sterilization apparatus further comprises a sterilization unit configured to inject a sterilization agent into a sterilization zone of the isolation chamber.

In order to improve the efficiency of the sterilization process the receptacle closure are pre-heated prior to being exposed to the sterilization agent.

Furthermore, a typical sterilization apparatus comprises a drying device adapted to direct heated air to the receptacle closures for drying off the sterilization agent residues from the receptacle closures prior to exiting the isolation chamber, so as to feed a receptacle closure devoid of any residues of the sterilization agent to the capping apparatus.

A drawback of such known sterilization apparatuses, in particular the conveying device, resides in the lacking possibility of adopting its processing velocities in dependence of the processing conditions of the capping apparatus.

A further drawback of such known sterilization apparatuses is that due the increased temperatures of the receptacle closures, the receptacle closures are more susceptible to deformations due to the forces acting within the succession of receptacle closures.

EP2500296A1 discloses a device for transporting articles, wherein the transport device is moved transversely in the transport direction during the feed stream of articles.

JPH04242525A discloses a method and a device for sterilizing container lids by irradiation while the lids are pushed by means of pushers attached to a chain. In particular, the document discloses a sterilization apparatus according to the preamble of claim 1.

Therefore, it is the desire to further improve the known sterilization apparatuses.

### DISCLOSURE OF INVENTION

It is an object of the present invention to provide in a straightforward and low-cost manner an improved sterilization apparatus for receptacle closures for overcoming at least one of the above-mentioned drawbacks.

According to the present invention, there is provided a sterilization apparatus according to claim 1.

Further advantageous embodiments of the sterilization apparatus are specified in the dependent claims.

### BRIEF DESCRIPTION OF THE DRAWINGS

Three embodiments will be described by way of example with reference to the accompanying drawings, in which:
Figure 1 is a schematic representation of a treatment machine, with parts removed for clarity;
Figure 2 is another schematic representation of a receptacle closure sterilization apparatus of the treatment machine of Figure 1, with parts removed for clarity;
Figure 3 is a perspective view of the sterilization apparatus of Figures 1 and 2 according to a first embodiment which is not according to the invention, with parts removed for clarity;
Figure 4 is a schematic side view of the sterilization apparatus of Figure 3, with parts removed for clarity;
Figure 5a is a sectionized view of details of the sterilization apparatus of Figures 3 and 4 according to a first variant, with parts removed for clarity;
Figure 5b is a sectionized schematic view of details of the sterilization apparatus of Figures 3 and 4 according to a second variant, with parts removed for clarity;
Figure 6 is perspective view of the sterilization apparatus of Figures 1 and 2 according to a second embodiment, which is according to the invention, and with parts removed for clarity;
Figure 7 is a sectionized view of details of the sterilization apparatus of Figure 6, with parts removed for clarity;
Figure 8 is a perspective view of the sterilization apparatus of Figures 1 and 2 according to a third embodiment which is not according to the invention, with parts removed for clarity; and
Figure 9 is a sectionized view of details of the sterilization apparatus of Figure 8, with parts removed for clarity.

### BEST MODES FOR CARRYING OUT THE INVENTION

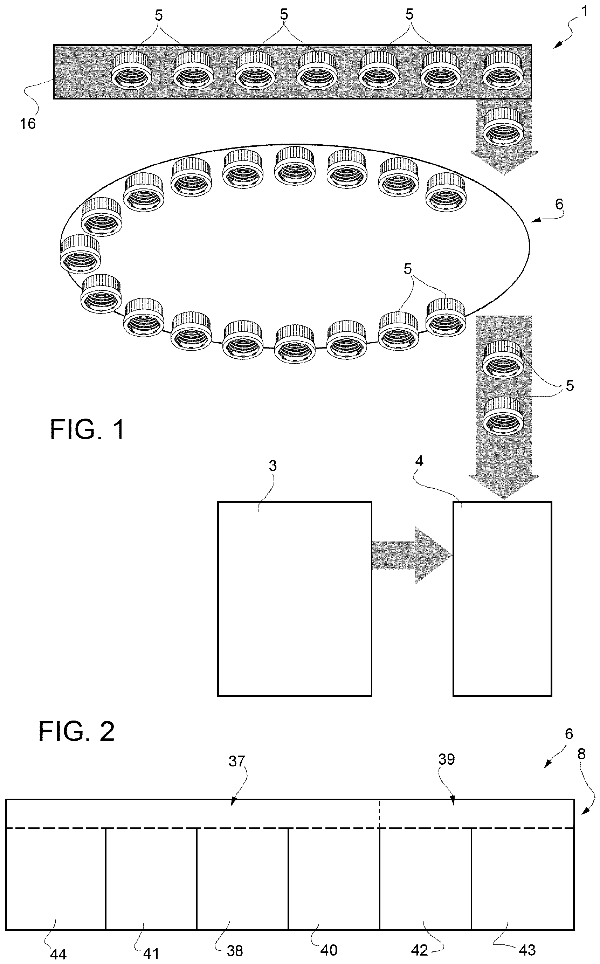

With particular reference to Figure 1, reference number 1 indicates as a whole a treatment machine for at least filling and capping receptacles, such as bottles, jars, vessels, containers or the like, in particular being made of base components, like glass, paper or cardboard, plastics, aluminum, steel, and composites.

Treatment machine 1 comprises at least:
- a filling apparatus 3 (known as such and not described in detail) for filling the receptacles with a pourable product, in particular a pourable food product such as carbonated liquids (e.g. sparkling water, soft drinks and beer) or non-carbonated liquids (e.g. still water, juices, teas, sport drinks, liquid cleaners, wine, emulsions, suspensions, high viscosity liquids and beverages containing pulps);
- a capping apparatus 4 (known as such and not described in detail) for applying at least one respective receptacle closure 5 to each one of the filled receptacles; and
- a sterilization apparatus 6 for sterilizing the receptacle closures 5 prior to being fed, in use, to capping apparatus 4.

Receptacle closures 5 may be of the type known as crown corks, screw caps, sports caps, stoppers or similar, and they may be produced from a variety of materials such as plastics, cork and metal. It is further known that the receptacle closures 5 can vary in format.

In the example shown in Figure 1, the specific type of receptacle closures 5 are threaded receptacle closures 5, in particular plastic screw caps, to be screwed onto the receptacles. However, it must be clear that the present invention may be also used to particular advantage for any other type of receptacle closures 5 such as crown corks, sports caps, stoppers and others made also from materials different than plastic, such as metal or cork.

According to a non-limiting embodiment not shown, treatment machine 1 could also comprise a molding apparatus for molding the receptacles from preforms.

With particular reference to Figures 2 to 4, sterilization apparatus 6 comprises:
- a conveying device 7 configured to advance receptacle closures 5 along an advancement path P;
- an isolation chamber 8 housing at least a portion of conveying device 7 so that at least a portion of advancement path P lies within isolation chamber 8; and
- a sterilization unit (not specifically shown) configured to sterilize receptacle closures 5 during advancement of the receptacle closures 5 along a sterilization portion P1 of advancement path P.

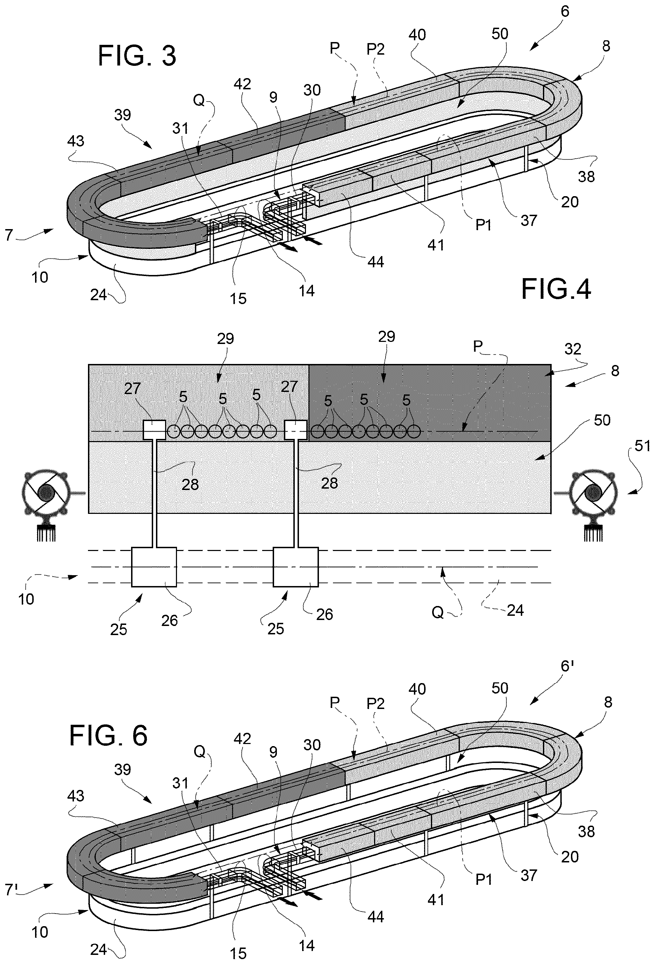

With particular reference to Figures 3 to 5a, conveying device 7 comprises at least:
- one guide track 9 defining and/or delimiting advancement path P; and
- an advancement unit 10 adapted to advance receptacle closures 5 along advancement path P and/or guide track 9.

According to a preferred non-limiting embodiment, guide track 9 comprises an inlet 14 for receiving receptacle closures 5 and an outlet 15 allowing for the outlet of receptacle closures 5 from guide track 9, in particular to capping apparatus 4. In particular, advancement path P extends between inlet 14 and outlet 15; i.e. conveying device 7 is configured to advance receptacle closures 5 between inlet 14 and outlet 15.

Preferentially but not necessarily, conveying device 7 also comprises an inlet conveying unit 16 (not shown in detail and known as such) configured to feed receptacle closures 5 to guide track 9, in particular inlet 14.

According to a preferred non-limiting embodiment, inlet conveying unit 16 or guide track 9 comprises an accumulation portion configured to allow for the accumulation of receptacle closures 5 prior to their advancement along advancement path P.

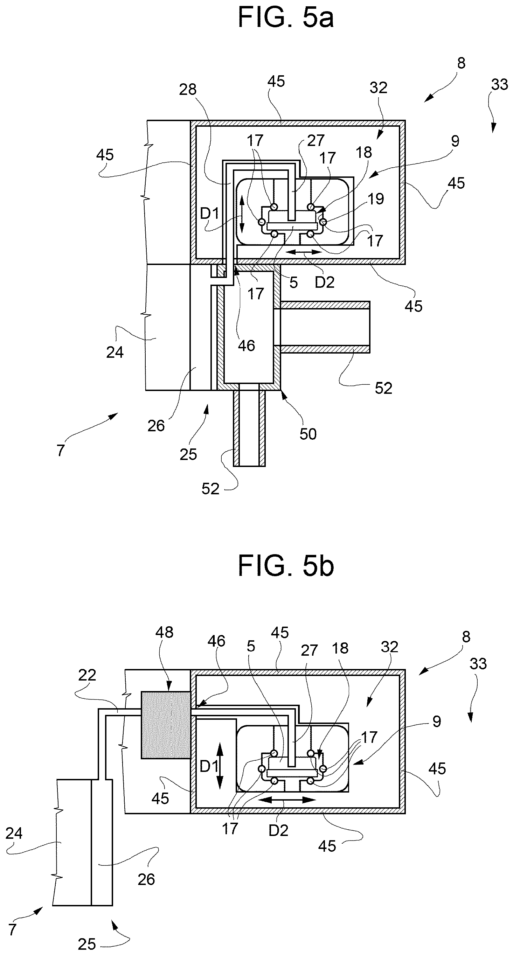

With particular reference to Figures 3 and 5a, guide track 9 comprises a plurality of guide bars 17, in the example embodiment shown six, for supporting and/or guiding receptacle closures 5, in particular during advancement along advancement path P.

Preferentially but not necessarily, guide track 9, in particular guide bars 17, delimit(s) and/or define(s) an advancement space 18 within which receptacle closures 5 advance, in use, along advancement path P.

Preferentially but not necessarily, guide bars 17 delimit the possible movement of receptacle closures 5 into a first direction D1 transversal, in particular orthogonal, to advancement path P and into a second direction D2 transversal, in particular orthogonal, to advancement path P and first direction D1. In particular, guide bars 17 (longitudinally) extend parallel to advancement path P.

In particular, in use, receptacle closures 5 are arranged in and/or advance in succession on guide track 9 and/or within advancement space 18 along advancement path P.

Preferentially but not necessarily, guide track 9 also comprises a support assembly 19 (having a plurality of support elements) for supporting guide bars 17.

According to a preferred non-limiting embodiment, guide track 9 comprises a plurality of linear and curved portions so as to define linear and curved portions of advancement path P.

According to a preferred non-limiting embodiment, at least a portion of guide track 9 is and/or of each guide bar 17 are arranged within isolation chamber 8.

According to a preferred non-limiting embodiment, conveying device 7 also comprises a support structure 20 carrying guide track 9 and/or isolation chamber 8.

With reference to Figures 4 and 5a, advancement unit 10 comprises at least:
- an endless track 24, in particular positioned adjacent to guide track 9 and/or isolation chamber 8;
- a plurality of cart assemblies 25 moveably coupled to endless track 24 and configured to interact with one or more receptacle closures 5 coupled to guide track 9; and
- an actuation assembly (not specifically shown) configured to advance and/or to control advancement of each cart assembly 25 along endless track 24 for advancing receptacle closures 5 along advancement path P.

As shown in Figures 3 to 5a, endless track 24 is arranged outside of isolation chamber 8.

With reference to Figures 4 and 5a, each cart assembly 25 comprises a cart 26 moveably coupled to endless track 24 and at least an engagement element 27 coupled to the respective cart 26 and configured to engage with one or more receptacle closures 5 for advancing the one or more receptacle closures 5 along advancement path P, in particular upon, in use, movement of the respective cart 26 along endless track 24. In particular, each engagement element 27 is configured to enter, in use, into advancement space 18.

Each engagement element 27 is configured to engage with a group 29 of receptacle closures 5 successively arranged on guide track 9, in particular so as to advance the respective group 29 along advancement path P upon advancement of the respective cart 26 along endless track 24 (and in consequence of the advancement of the respective engagement element 27).

Each engagement element 27 is configured to contact at least the receptacle closure 5 of the respective group 29, which is arranged upstream from the others along advancement path P, so as to apply a pushing force on the respective group 29 upon advancement of the respective cart 26 along endless track 24. According to a preferred non-limiting embodiment, each group 29 comprises not more than 30, in particular between 5 to 20, even more particular between 5 to 15, receptacle closures 5. In particular, this allows to reduce the forces acting on receptacle closures 5, thereby e.g. limiting the risk of deforming receptacle closures 5.

Preferentially but not necessarily, each cart assembly 25 also comprises a coupling structure 28 having a first portion carrying and/or supporting the respective engagement element 27 and a second portion opposed to the first portion and being connected to the respective cart 26, in particular for mechanically coupling the respective engagement element 27 to the respective cart 26.

According to a preferred non-limiting embodiment, the actuation unit is configured to advance each cart assembly 25, in particular each cart 26 and/or each engagement element 27, along an endless advancement path Q defined by endless track 24.

In particular, path Q is parallel to advancement path P at least between inlet 14 and outlet 15.

The actuation assembly is configured to control the advancement, in particular the respective advancement speeds, of each cart assembly 25, in particular each cart 26, along endless track 24, independently from the others. In particular, the actuation assembly is also configured to control and/or vary the advancement speed of each cart assembly 25, in particular the respective cart 26, during advancement along endless track 24; i.e. the advancement speed is not necessarily constant during advancement of the respective cart 26 along endless track 24 and the respective cart 26 can be subject to accelerations and decelerations. Preferentially but not necessarily, the advancement speed of each cart assembly 25 is, in use, selectively controlled in dependence of the local position of each cart assembly 25 on endless track 24.

Preferentially but not necessarily, the actuation assembly comprises a plurality of coils (not shown and known as such) for selectively forming an electromagnetic field and being positioned (adjacent and) along endless track 24 and each cart assembly 25 comprises at least one interaction element (not shown), e.g. of the magnetic or ferromagnetic type, configured to interact with the electromagnetic field. In particular, in use, the selective control of the coils allows to selectively control the advancement, e.g. the advancement speed, of each one of cart assemblies 25 along endless track 24 independently from the other cart assemblies 25.

According to a preferred non-limiting embodiment, endless track 24 comprises a first mechanical coupling portion (not specifically shown) and each cart 26 comprises a second mechanical coupling portion (not specifically shown) for mechanically mounting each cart 26 onto endless track 24, in particular for determining and/or defining precisely advancement path Q. E.g. the respective second mechanical coupling portion of each cart 26 could comprise a plurality of running wheels configured to run along a surface profile of the first mechanical coupling portion.

According to a preferred non-limiting embodiment, conveying device 7 comprises at least one parking track (not shown) coupled and/or connected to endless track 24 and being configured to store one or more cart assemblies 25 and to allow the addition and/or removal of cart assemblies 25 to and/or from endless track 24, in particular in dependence of processing conditions (such as the number of receptacle closures 5 to be processed per hour and/or the number of receptacles to be capped per hour and/or the number of receptacle closures 5 to be sterilized per hour) of treatment machine 1 and/or capping apparatus 4 and/or sterilization apparatus 6.

E.g. in the case of the overall production capacity being reduced or increased with respect to a previous production capacity it is possible to direct one or more cart assemblies 25 respectively from endless track 24 to the parking track and from the parking track to endless track 24. Such a control can e.g. be done during and/or before and/or after a production cycle.

In particular, the parking track also allows to mount new cart assemblies 25 or to dismount used cart assemblies 25 (e.g. requiring a maintenance step) without interrupting the overall production.

With reference to Figures 3 to 5, isolation chamber 8 is configured to house at least a portion of guide track 9.

Preferentially but not necessarily, guide track 9 comprises an initial portion 30 having inlet 14 and a main portion 31 having outlet 15. At least main portion 31 is arranged within isolation chamber 8.

According to a preferred non-limiting embodiment, isolation chamber 8 is configured to separate an inner environment 32 from an outer environment 33.

According to a preferred non-limiting embodiment, sterilization apparatus 6 also comprises a conditioning unit fluidically connected to isolation chamber 8 and configured to control an atmosphere within isolation chamber 8 and/or of inner environment 32. In particular, the conditioning unit is configured to at least locally control the pressure within isolation chamber 8 and/or the temperature and/or the humidity and/or the sterility and/or the cleanliness of the atmosphere.

Isolation chamber 8 comprises at least a sterilization zone 37 within which receptacle closures 5 advance along sterilization portion P1. In use, receptacle closures 5 are sterilized during advancement along sterilization portion P1. Even more particular, the sterilization unit is configured to inject a sterilization agent, such as hydrogen peroxide and/or any other chemical sterilization agent in gaseous, vaporous and/or liquid form, into at least an injection portion 38 of sterilization zone 37. Preferentially but not necessarily, in use, a cloud of sterilization agent develops within at least injection portion 38.

According to a preferred non-limiting embodiment, isolation chamber 8 also comprises an aseptic zone 39 arranged downstream of sterilization zone 37. In particular, aseptic zone 39 receives, in use, sterilized receptacle closures 5 from sterilization zone 37. Even more particular, in use, sterilized receptacle closures 5 advance within aseptic zone 39 along a sterilized portion P2 of advancement path P, in particular by advancement of the respective engagement elements 27.

In particular, outlet 15 is arranged within aseptic zone 39.

According to a preferred non-limiting embodiment, the conditioning unit is configured to control at least a first pressure within sterilization zone 37 higher than the ambient pressure and, preferentially but not necessarily at least a second pressure within aseptic zone 39 being higher than the first pressure. In this manner, it is guaranteed to avoid the entrance of contaminations from sterilization zone 37 into aseptic zone 39 and from outer environment 33 into sterilization zone 37.

According to a preferred non-limiting embodiment, sterilization zone 37 also comprises a contact portion 40 arranged downstream from injection portion 38. In particular, in use, during advancement of receptacle closures 5 within contact portion 40 the sterilization agent in contact with (having been deposited onto) receptacle closures 5 act on receptacle closures 5. In particular, the concentration of the sterilization agent within contact portion 40 is significantly lower than the one within injection portion 38.

According to a preferred non-limiting embodiment, sterilization apparatus 6 comprises a pre-heating unit (not shown and known as such) configured to pre-heat receptacle closures 5 during, in use, advancement within a pre-heating portion 41 of sterilization zone 37, pre-heating portion 41 being arranged upstream of injection portion 38 along advancement path P. In particular, the pre-heating unit is configured to direct a heated sterile gas, in particular heated sterile air into pre-heating portion 41. In this way, it is possible to improve the sterilization efficiency of receptacle closures 5, in particular by improving the activity of the sterilization agent getting, in use, into contact with receptacle closures 5.

With particular reference to Figures 2 and 3, aseptic zone 39 comprises a drying and venting portion 42. In particular, during advancement of receptacle closures 5 within drying and venting portion 42 the sterilization agent dries of and/or evaporates from receptacle closures 5.

Preferentially but not necessarily, sterilization apparatus 6 comprises a venting unit (not shown and known as such) configured to vent and/or extract gas from drying and venting portion 42. In particular, the venting unit is configured to remove any residues of the sterilization agent being present within drying and venting portion 42.

In particular, by providing for drying and venting portion 42 it is possible to guarantee that receptacle closures 5 being finally applied onto the receptacles are substantially void of any residues of the sterilization agent.

According to a preferred non-limiting embodiment, aseptic zone 39 also comprises a cooling portion 43 configured to allow an active and/or passive cooling of receptacle closures 5 during, in use, advancement within cooling portion 43, in particular so that the temperature of receptacle closures 5 ranges between 20 °C and 60 °C, in particular between 20°C and 40°C, during transfer of receptacle closures 5 to capping apparatus 4 and/or during application of receptacle closures 5 on the respective receptacles.

According to a preferred non-limiting embodiment, cooling portion 43 is arranged downstream from drying and venting portion 42 along advancement path P.

It must be understood that each engagement element 27 advances, in use, through sterilization zone 37, in particular injection portion 38 and/or contact portion 40 and/or pre-heating portion 41, and/or aseptic zone 39, in particular within drying and venting portion 42 and/or cooling portion 43, for advancing the respective receptacle closures 5 through respectively sterilization zone 37, in particular injection portion 38 and/or contact portion 40 and/or pre-heating portion 41, and/or aseptic zone 39, in particular within drying and venting portion 42 and/or cooling portion 43.

According to a preferred non-limiting embodiment, sterilization apparatus 6 also comprises an auxiliary sterilization unit (not shown) configured to sterilize engagement elements 27 while advancing within an auxiliary sterilization portion 44 of sterilization zone 37, in particular being arranged upstream of injection portion 38 (and pre-heating portion 41) along advancement path P. In particular, the sterilization of engagement elements 27 guarantees that the engagement elements 27 do not contaminate the respective receptacle closure 5 with which engagement elements 27 get, in use, directly into contact.

Preferentially but not necessarily, the auxiliary sterilization unit is configured to sterilize engagement elements 27 by means of physical and/or chemical sterilization.

According to a preferred non-limiting embodiment, the actuation assembly is configured to selectively control the advancement, in particular the advancement speed, of cart assemblies 25, in particular of carts 26, for selectively controlling the advancement, in particular the advancement speed, of the respective engagement element 27 within sterilization zone 37, in particular within injection portion 38 and/or contact portion 40 and/or pre-heating portion 41, and/or aseptic zone 39, in particular within drying and venting portion 42 and/or cooling portion 43, in function of the type of receptacle closure and/or of the type of sterilization and/or of the type and the physical form of the sterilization agent and/or of the processing conditions (such as the number of receptacle closures 5 to be processed per hour and/or the number of receptacles to be capped) of treatment machine 1 and/or capping apparatus 4. In this manner it is possible to selectively control the permanence time of receptacle closures 5 within sterilization zone 37, in particular within injection portion 38 and/or contact portion 40 and/or pre-heating portion 41, and/or aseptic zone 39, in particular within drying and venting portion 42 and/or cooling portion 43.

In comparison to the state-of-the-art sterilization apparatuses this is advantageous as these only allow for one advancement speed equal for the complete succession of receptacle closures 5 supported and/or guided by guide track 9. Another advantage with respect to the known sterilization apparatuses resides in the fact that the advancement of receptacle closures 5 can be interrupted and the receptacle closures 5 can be recovered afterwards, as it is possible to place these in portions of isolation chamber 8, which allow receptacle closures 5 to retain their form. This is not possible for the known sterilization apparatuses. E.g. in this latter case, the interruption of the advancement of the receptacle closures would mean that a certain quantity would be exposed to increased temperatures for times leading to the deterioration and/or the deformation of the receptacle closures rendering these unusable further on.

With particular reference to Figures 2 and 5a, isolation chamber 8 is realized as an isolation channel having an inlet portion and an outlet portion for respectively allowing the infeed and outfeed of receptacle closures 5 respectively into and out of isolation chamber 8.

According to a preferred non-limiting embodiment, comprises a plurality of delimiting walls 45 isolation chamber 8 and delimiting the inlet portion and the outlet portion.

According to a preferred non-limiting embodiment, isolation chamber, in particular at least one of the delimiting walls 45, comprises an inlet slit 46 configured to allow the entrance of the respective first portion of each coupling structure 28 into isolation chamber 8. In particular, by providing for inlet slit 46 it is possible to establish the mechanical coupling of each engagement element 27, which at least partially advances within isolation chamber 8, and the respective cart 26, which advances outside of isolation chamber 8.

Preferentially but not necessarily, sterilization apparatus 6 comprises a suction channel 50 extending parallel to and enclosing inlet slit 46 and sterilization apparatus 6, in particular the conditioning unit, comprises an aspiration unit 51 configured to apply a suction force within suction channel 50 so as to create and/or guarantee a flow of fluids (gases and/or liquids) from isolation chamber 8 into suction channel 50.

Preferentially but not necessarily, the conditioning unit, in particular aspiration unit 51, is configured to control a third pressure within suction channel 50, the third pressure being lower than the first pressure and the second pressure.

According to a preferred non-limiting embodiment, suction channel 50 comprises at least one outlet duct 52 allowing for the evacuation of fluids present within suction channel 50. According to the non-limiting embodiment shown, suction channel 50 comprises at least two outlet ducts 52, one for the evacuation of liquids and the other one for the evacuation of gases.

According to a variant, not according to the invention, of sterilization apparatus 6 partially shown in Figure 5b, sterilization apparatus 6 does not comprise a suction channel 50 and does not comprise aspiration unit 51. Instead, sterilization apparatus 6 comprises a liquid seal 48 extending parallel to and enclosing inlet slit 46. In particular, liquid seal 48 separates inner environment 32 from outer environment 33 and is configured to guarantee that no contaminations may enter into isolation chamber 8 and/or inner environment 32 while allowing the entrance of coupling structures 28 into isolation chamber 8.

According to a preferred non-limiting embodiment of the variant shown in Figure 5b, liquid seal 48 comprises a channel extending parallel to and enclosing inlet slit 46 and being at least partially filled with a liquid, in particular a sterile and/or sterilization liquid. In particular, a portion of coupling structure 28 extends within liquid seal 48.

In use, treatment machine 1 fills receptacles with the pourable product and applies receptacle closures 5 onto the filled receptacles.

In more detail, operation of treatment machine 1 comprises at least the steps of:
- filling, in particular by filling apparatus 3, the receptacles with the pourable product;
- applying receptacle closures 5, in particular by capping apparatus 4, onto the filled receptacles; and
- sterilizing receptacle closures 5, in particular by sterilization apparatus 6, prior to their application on the receptacles.

More precisely, the step of sterilizing receptacle closures 5 comprises at least the (sub-)steps of:
- advancing receptacle closures 5 along advancement path P; and
- sterilizing receptacle closures 5 during advancement along sterilization portion P1.

Advantageously, the step of advancing comprises at least the (sub-)step of coupling receptacle closures 5 to guide track 9.

According to a preferred non-limiting embodiment, during the step of coupling, a plurality of receptacle closures 5 are arranged on guide track 9 in succession so as to form a respective group 29.

Preferentially but not necessarily, receptacle closures 5 are feed to guide track 9, in particular by inlet conveying unit 16.

Advantageously, the step of advancing also comprises the (sub-)steps of:
- engaging one respective engagement element 17 of a respective cart assemblies 25 with at least one receptacle closure 5, preferentially with the receptacle closure 5 being the most upstream one of the respective group 29 along advancement path P; and
- controlling the advancement of cart assemblies 25, in particular carts 26, along endless track 24 for advancing the respective receptacle closure 5 and/or the respective group 29 along advancement path P by advancing the respective engagement element 17 (the one engaging with the receptacle closure 5 and/or the most upstream receptacle closure 5 of the respective group 29).

According to a preferred non-limiting embodiment, during the step of controlling, the advancement speed of each cart assembly 25, in particular the respective cart 26, is controlled in dependence of the processing conditions of treatment machine 1 and/or capping apparatus 4 and/or sterilization apparatus 6 itself and/or of the type of receptacle closure.

Preferentially but not necessarily, during the step of controlling, the advancement speed of each cart assembly 25, in particular the respective cart 26, is locally controlled; i.e. the advancement speed may be varied during advancement along advancement path P.

In alternative or in addition, during the step of controlling, the advancement speed of each cart assembly 25 is controlled in dependence of the processing conditions of sterilization apparatus 6. Preferentially but not necessarily, the advancement speed of each cart assembly 25 is controlled in dependence of the local position of receptacle closures 5 within isolation chamber 8, in particular such to control the residence time within sterilization zone 37, in particular injection portion 38 and/or contact portion 40 and/or pre-heating portion 41, and/or aseptic zone 39, in particular within drying and venting portion 42 and/or cooling portion 43. Even more particular, such a control is also made in dependence of the type of receptacle closure. In this manner, it is possible to optimize the sterilization of receptacle closures 5. E.g. when comparing a sports cap with a screw cap, it is evident that the geometry between both differs and, accordingly, the most suited residence times within the varying portions of isolation chamber 8 a different. Conveying device 7 allows to precisely control these residence time.

Preferentially but not necessarily, the number of cart assemblies 25 being arranged on endless track 24 is a function of the processing conditions and/or the type of receptacle closure.

According to a preferred non-limiting embodiment, during the step of advancing, the plurality of coils selectively generates an electromagnetic field for selectively controlling advancement of cart assemblies 25.

Preferentially but not necessarily, the step of advancing (i.e. the operation of conveying device 7) also comprises the (sub-)steps of adding or removing cart assemblies 25, during which cart assemblies 25 are added or removed onto endless track 24, in particular from or to the parking track. It should be mentioned that the step of adding or removing can be executed also prior or after operation of treatment machine 1 and/or capping apparatus 4 and/or sterilization apparatus 6.

According to a preferred non-limiting embodiment, the step of sterilization also comprises at least:
- a step of evacuation during which fluids are evacuated from isolation chamber 8, in particular into and out of suction channel 50; and/or
- a step of conditioning, during which an atmosphere within isolation chamber 8 is controlled.

It must be understood that the step of sterilization does not require the step of evacuation in the case of using sterilization apparats 6 according to the variant of Figure 5b.

Preferentially but not necessarily, during the step of conditioning at least the pressure and/or the temperature and/or the sterility and/or the humidity is controlled within isolation chamber 8. In particular, during the step of conditioning at least the first pressure and the second pressure are controlled such that the first pressure is larger than the ambient pressure and the second pressure is larger than the first pressure. Even more particular, also the third pressure is controlled to be smaller than the first pressure.

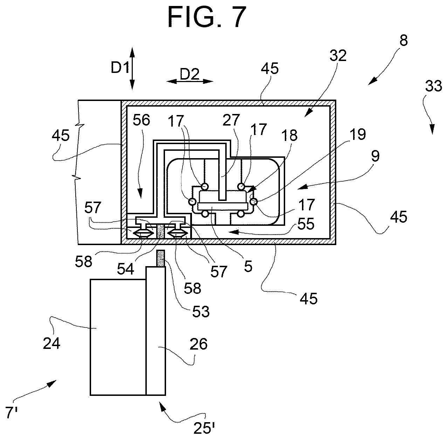

With reference to Figures 6 and 7, number 6' indicates an alternative embodiment of a sterilization apparatus which is according to the present invention; as sterilization apparatus 6' is similar to sterilization apparatus 6, the following description is limited to the differences between them, and using the same references, where possible, for identical or corresponding parts.

Sterilization apparatus 6' differs from sterilization apparatus 6 in comprising conveying device 7'.

In particular, sterilization apparatus 6' also differs from sterilization apparatus 6 in not comprising suction channel 50 and aspiration unit 51 and in that isolation chamber 8 does not comprise inlet slit 46.

With reference to Figures 6 and 7, conveying device 7' is similar to conveying device 7 and for that reason, the following description is limited to the differences between them, and using the same references, where possible, for identical or corresponding parts.

Conveying device 7' comprises a plurality of cart assemblies 25' instead of cart assemblies 25.

As cart assemblies 25' are similar to cart assemblies 25 the following description is limited to the differences between them, and using the same references, where possible, for identical or corresponding parts.

Cart assemblies 25' differ from cart assemblies 25 in that the respective engagement elements 27 and the respective carts 26 are not mechanically coupled to one another, but by means of magnetic and/or electromagnetic coupling. In this way it is possible to transfer, in use, the movement of carts 26 along endless track 24 to engagement elements 27 and in consequence to the respective receptacle closures 5.

According to the invention, each cart assembly 25 comprises at least a first interaction member 53, e.g. a first magnet, coupled and/or connected to the respective cart 26 and a second interaction member 54, e.g. a second magnet, coupled and/or connected to the respective engagement element 27.

Each first interaction member 53 and the respective second interaction member 54 are configured to interact with one another by means of magnetic and/or electromagnetic interaction so as to transfer a movement of the respective cart 26 to the respective engagement element 27.

Preferentially but not necessarily, each first interaction member 53 and the respective second interaction member 54 are adjacent and spaced apart from one another. In particular, in use, when the respective engagement element 27 advances within isolation chamber 8, each first interaction member 53 and the respective second interaction member 54 interpose one of the delimiting walls 45 between one another.

According to a preferred non-limiting embodiment, conveying device 7' comprises a guiding assembly 55 configured to guide the advancement of engagement elements 27 and, in particular at least a portion of guiding assembly 55 being mounted to (an inner surface of) isolation chamber 8, even more particular to one of delimiting walls 45.

According to a preferred non-limiting embodiment, each cart assembly 25' comprises a guiding structure 56 coupled to and configured to interact with guiding assembly 55. In particular, each guiding structure 56 carries the respective second interaction member 54 and the respective engagement element 27, even more particular at opposing ends of guiding structure 56.

Preferentially but not necessarily, guiding assembly 55 comprises at least one rail 57, in particular a plurality of rails 57, extending substantially parallel to guide track 9 and configured to interact with and to guide each guiding structure 56.

Preferentially but not necessarily, each guide structure 56 comprises one or more guide wheels 58 configured to run, in use, along at least one guide rail 57.

According to the non-limiting embodiment shown in Figure 7, each guide wheel 58 is interposed between two respective guide rails 57.

The function of sterilization apparatus 6' and of conveying device 7' is similar to the function of respectively sterilization apparatus 6 and conveying device 7. For this reason, we solely refer to the differences between the function of sterilization apparatus 6' and sterilization apparatus 6 and of the function of conveying device 7' and conveying device 7.

A main difference resides in the operation of conveying device 7' with respect to the one of conveying device 7. The difference lies in that carts 26 do not transfer their advancement to the respective engagement elements 27 by means of a mechanical coupling, but by means of magnetic and/or electromechanical coupling.

In particular, operation of sterilization apparatus 6' also does not require a step of evacuation of fluids from a suction channel as sterilization apparatus 6' does not comprise such a suction channel.

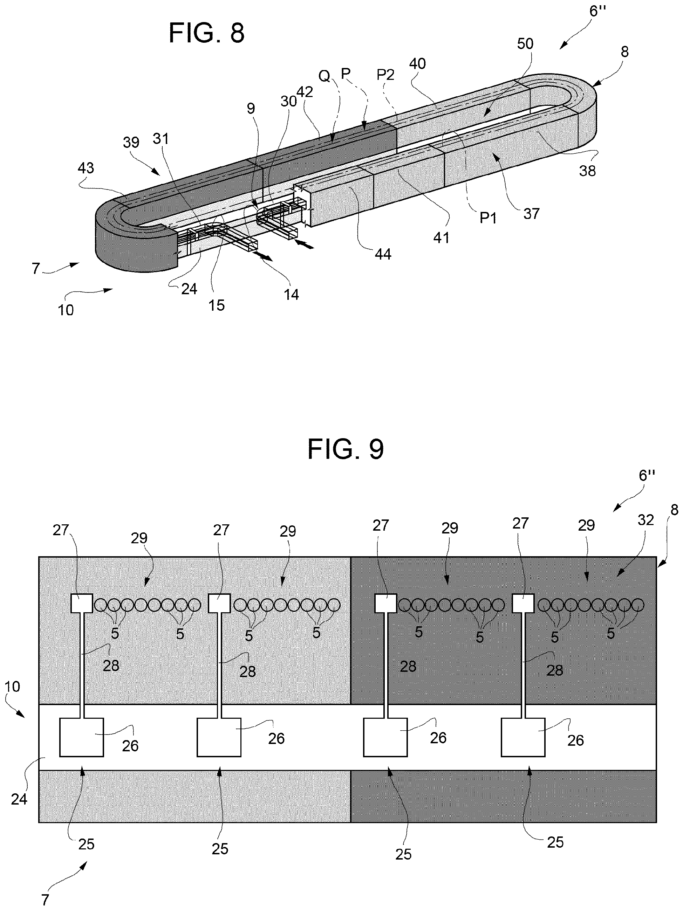

With reference to Figures 8 and 9, number 6" indicates an alternative embodiment of a sterilization apparatus not according to the present invention; as sterilization apparatus 6" is similar to sterilization apparatus 6, the following description is limited to the differences between them, and using the same references, where possible, for identical or corresponding parts.

In particular, sterilization apparatus 6" differs from sterilization apparatus 6 in that the dimension of isolation chamber 8 is such that endless track 24 is arranged within isolation chamber 8. This means that, in use, each cart 26 advances together with the respective engagement element 27 within isolation chamber 8.

Preferentially but not necessarily, the auxiliary sterilization unit is also configured to sterilize carts 26.

The function of sterilization apparatus 6" is similar to the function of sterilization apparatus 6. For this reason, we solely refer to the differences between the function of sterilization apparatus 6".

In particular, according to a preferred non-limiting embodiment, during the step of auxiliary sterilization, also carts 26 are sterilized.

The advantages of sterilization apparatus 6' according to the present invention will be clear from the foregoing description.

In particular, conveying devices 7 and 7' allow for a more flexible advancement of receptacle closures 5. In particular, by providing for a plurality of respectively cart assemblies 25 and cart assemblies 25', which can be individually controlled, it is possible to advance a respective group 29 of receptacle closures 5 independently from the other groups 29. It is e.g. possible to increase or decrease the advancement speed so as to react on the number of receptacle closures 5 needed by capping apparatus 4.

It is a further advantage that it is possible to modify and/or control the capacity of conveying devices 7 and 7' by varying the number of cart assemblies 25 and 25' advancing along endless track 24.

Another advantage resides in that the forces acting on the single receptacle closures 5 are decreased with respect to the state-of-the-art, decreasing the risk of deforming receptacle closures 5 during their advancement along advancement path P, as the number of receptacle closures 5 pushing against one another is reduced with respect to the state-of-the art, in which receptacle closures 5 advance along the guide track by exerting a force on the most upstream receptacle closure 5, which is then transferred on the other receptacle closures.

An even further advantage resides in the possibility to selectively control the residence time of receptacle closures 5, in particular of the respective group 29, within the varying portions of isolation chamber 8.

Clearly, changes may be made to sterilization apparatus 6' without departing from the scope of protection as defined in the accompanying claims.

According to an embodiment not according to the invention, sterilization apparatus 6" could comprise conveying device 7' instead of conveying device 7.

According to a non-limiting alternative embodiment not shown, each engagement element 27 could be configured to carry a plurality of receptacle closures 5 and guide track 9 could be configured to laterally delimit receptacle closures 5. In particular, according to such an alternative embodiment, each engagement element 27 could have a support portion configured to receive and carry a respective group of receptacle closures 5.

According to a non-limiting alternative embodiment not shown, conveying apparatuses 7 and 7' could comprise a plurality of guide tracks 9 of which each one is configured to receive certain types and/or formats of receptacle closures different from the ones of the other guide tracks 9. In particular, each one of the respective cart assemblies 25 and 25' is configured to advance receptacle closures 5 along and/or within each one of the plurality of guide tracks 9.

## Claims

1. A sterilization apparatus (6') for sterilizing receptacle closures (5), the apparatus (6') comprising a conveying device (7') for advancing receptacle closures (5) along an advancement path (P), the conveying device (7') comprising:
- at least one guide track (9) defining and/or delimiting the advancement path (P); and
- an advancement unit (10) adapted to advance the receptacle closures (5) along the advancement path (P);
wherein the advancement unit (10) comprises:
- an endless track (24);
- a plurality of cart assemblies (25') moveably coupled to the endless track (24) and configured to interact with one or more receptacle closures (5) coupled to the guide track (9);
- an actuation assembly configured to control advancement of the cart assemblies (25') along the endless track (24) for advancing the receptacle closures (5) along the advancement path (P);
wherein each cart assembly (25') comprises a cart (26) moveably coupled to the endless track (24) and at least an engagement element (27) coupled to the respective cart (26) and configured to engage with one or more receptacle closures (5) for advancing the one or more receptacle closures (5) along the advancement path (P);
wherein the apparatus comprises an isolation chamber (8) housing at least a portion of the guide track (9) and comprising at least a sterilization zone (37) within which the receptacle closures (5) advancing along a sterilization portion (P1) of the advancement path (P) are, in use, sterilized; and
- wherein the apparatus comprises a sterilization unit configured to sterilize the receptacle closures (5) during advancement of the receptacle closures (5) along the sterilization portion (P1);
wherein the respective engagement element (27) of each cart assembly (25') is arranged within the isolation chamber (8) at least during advancement of the respective receptacle closures (5) within the sterilization zone (37);
wherein the guide track (9) is configured to support and/or guide the receptacle closures (5) during advancement along the advancement path (P);
**characterized in that**:
the endless track (24) is arranged outside of the isolation chamber (8);
the actuation assembly is configured to control each cart assembly (25') independently from the others;
each cart assembly (25') comprises:
- a first interaction member (53) coupled to the respective cart (26); and
- a second interaction member (54) coupled to the respective engagement element;
each first interaction member (53) and the respective second interaction member (54) are configured to interact with one another by means of magnetic and/or electromagnetic interaction so as to transfer a movement of the respective cart (26) to the respective engagement element (27);
each engagement element (27) is configured to engage with a group (29) of receptacle closures (5) successively arranged on the guide track (9);
each engagement element (27) is configured to contact at least the receptacle closure (5) of the group (29) of receptacle closures (5), which is arranged upstream from the others along the advancement path (P), so as to apply a pushing force on the group (29) of receptacle closures (5) upon advancement of the respective cart (26) for advancing the group (29) of receptacle closures (5) along the advancement path (P).

2. The sterilization apparatus according to any one of the preceding claims, wherein the actuation assembly comprises a plurality of coils for selectively forming an electromagnetic field and being arranged along the endless track (24);
wherein each cart assembly (25') comprises at least one interaction element configured to interact with the electromagnetic field.

3. The sterilization apparatus according to any one of the preceding claims, wherein the conveying device comprises a parking track connected to the endless track (24) and adapted to store one or more cart assemblies (25'); wherein the parking track is configured to allow the addition or removal of cart assemblies (25') from the endless track (24), in particular in dependence of processing conditions of a treatment machine (1; 4; 6') within which the conveying device in (7') is integrated and/or to which the conveying device (7') is connected and/or to which the conveying device (7') feeds, in use, the receptacle closures (5).

4. The sterilization apparatus according to any one of the preceding claims, wherein the number and/or the advancement speeds of the cart assemblies (25') advancing, in use, along the endless track (24) is a function of processing conditions of a treatment machine (1; 4; 6') within which the conveying device (7') is integrated and/or to which the conveying device (7') is connected and/or to which the conveying device (7, 7') feeds, in use, the receptacle closures (5).

## Patentansprüche

1. Sterilisationsvorrichtung (6') zum Sterilisieren von Behälterverschlüssen (5), wobei die Vorrichtung (6') eine Fördervorrichtung (7') zum Vorschieben von Behälterverschlüssen (5) entlang eines Vorschubwegs (P) umfasst, wobei die Fördervorrichtung (7') umfasst:
- mindestens eine Führungsbahn (9), die den Vorschubweg (P) definiert und/oder begrenzt; und
- eine Vorschiebeeinheit (10), die zum Vorschieben der Behälterverschlüsse (5) entlang des Vorschubwegs (P) angepasst ist;
wobei die Vorschiebeeinheit (10) umfasst:
- eine Endlosbahn (24);
- eine Vielzahl von Wagenanordnungen (25'), die beweglich mit der Endlosbahn (24) verbunden sind und die dazu ausgelegt sind, mit einem oder mehreren, mit der Führungsbahn (9) verbundenen Behälterverschlüssen (5) zusammenzuwirken;
- eine Betätigungsanordnung, die zur Steuerung des Vorschubs der Wagenanordnungen (25') entlang der Endlosbahn (24) zum Vorschieben der Behälterverschlüsse (5) entlang des Vorschubwegs (P) ausgelegt ist;
wobei jede Wagenanordnung (25') einen Wagen (26), der beweglich mit der Endlosbahn (24) verbunden ist, und mindestens ein Eingriffselement (27) umfasst, das mit dem jeweiligen Wagen (26) verbunden und für einen Eingriff mit einem oder mehreren Behälterverschlüssen (5) zum Vorschieben des einen oder der mehreren Behälterverschlüsse (5) entlang des Vorschubwegs (P) ausgelegt ist;
wobei die Vorrichtung eine Isolierkammer (8) umfasst, in der mindestens ein Teil der Führungsbahn (9) untergebracht ist und die mindestens eine Sterilisationszone (37) umfasst, in der die Behälterverschlüsse (5), die entlang eines Sterilisationsabschnitts (P1) des Vorschubwegs (P) vorgeschoben werden, bei der Benutzung sterilisiert werden; und
- wobei die Vorrichtung eine Sterilisationseinheit umfasst, die zum Sterilisieren der Behälterverschlüsse (5) während des Vorschubs der Behälterverschlüsse (5) entlang des Sterilisationsabschnitts (P1) ausgelegt ist;
wobei das jeweilige Eingriffselement (27) jeder Wagenanordnung (25') mindestens während des Vorschubs der jeweiligen Behälterverschlüsse (5) in der Sterilisationszone (37) in der Isolierkammer (8) angeordnet ist;
wobei die Führungsbahn (9) zur Unterstützung und/oder Führung der Behälterverschlüsse (5) während des Vorschubs entlang des Vorschubwegs (P) ausgelegt ist;
**dadurch gekennzeichnet, dass**:
die Endlosbahn (24) außerhalb der Isolierkammer (8) angeordnet ist;
die Betätigungsanordnung zur Steuerung jeder Wagenanordnung (25') unabhängig von den anderen ausgelegt ist;
jede Wagenanordnung (25') umfasst:
- ein erstes Interaktionselement (53), das mit dem jeweiligen Wagen (26) verbunden ist; und
- ein zweites Interaktionselement (54), das mit dem jeweiligen Eingriffselement verbunden ist;
jedes erste Interaktionselement (53) und das jeweilige zweite Interaktionselement (54) dazu ausgelegt sind, durch magnetische und/oder elektromagnetische Interaktion zusammenzuwirken, um eine Bewegung des jeweiligen Wagens (26) auf das jeweilige Eingriffselement (27) zu übertragen;
jedes Eingriffselement (27) für einen Eingriff mit einer Gruppe (29) von Behälterverschlüssen (5), die nacheinander auf der Führungsbahn (9) angeordnet sind, ausgelegt ist;
jedes Eingriffselement (27) dazu ausgelegt ist, mindestens den Behälterverschluss (5) der Gruppe (29) von Behälterverschlüssen (5) zu berühren, der stromaufwärts von den anderen entlang des Vorschubwegs (P) angeordnet ist, um so eine Schubkraft auf die Gruppe (29) von Behälterverschlüssen (5) beim Vorschieben des jeweiligen Wagens (26) zum Vorschieben der Gruppe (29) von Behälterverschlüssen (5) entlang des Vorschubwegs (P) auszuüben.

2. Sterilisationsvorrichtung nach einem der vorhergehenden Ansprüche, wobei die Betätigungsanordnung eine Vielzahl von Spulen zum selektiven Bilden eines elektromagnetischen Feldes umfasst, und die entlang der Endlosbahn (24) angeordnet sind;
wobei jede Wagenanordnung (25') mindestens ein Interaktionselement umfasst, das zum Zusammenwirken mit dem elektromagnetischen Feld ausgelegt ist.

3. Sterilisationsvorrichtung nach einem der vorhergehenden Ansprüche, wobei die Fördervorrichtung eine Parkbahn umfasst, die mit der Endlosbahn (24) verbunden ist und zum Lagern von einer oder mehreren Wagenanordnungen (25') angepasst ist;
wobei die Parkbahn dazu ausgelegt ist, Hinzufügen oder Entfernen von Wagenanordnungen (25') von der Endlosbahn (24) zu erlauben, insbesondere in Abhängigkeit von Verarbeitungsbedingungen einer Behandlungsmaschine (1; 4; 6'), in der die Fördervorrichtung (7') integriert ist und/oder mit der die Fördervorrichtung (7') verbunden ist und/oder der die Fördervorrichtung (7') bei der Benutzung die Behälterverschlüsse (5) zuführt.

4. Sterilisationsvorrichtung nach einem der vorhergehenden Ansprüche, wobei die Anzahl und/oder die Vorschubgeschwindigkeiten der Wagenanordnungen (25'), die bei der Benutzung entlang der Endlosbahn (24) vorgeschoben werden, von den Verarbeitungsbedingungen einer Behandlungsmaschine (1; 4; 6'), in der die Fördervorrichtung (7') integriert ist und/oder mit der die Fördervorrichtung (7') verbunden ist und/oder der die Fördervorrichtung (7') bei der Benutzung die Behälterverschlüsse (5) zuführt, abhängig sind.

## Revendications

1. Appareil de stérilisation (6') pour stériliser des fermetures de récipient (5), l'appareil (6') comprenant un dispositif de transport (7') pour faire avancer des fermetures de récipient (5) le long d'un trajet d'avancement (P), le dispositif de transport (7') comprenant :
- au moins un chemin de guidage (9) définissant et/ou délimitant le trajet d'avancement (P) ; et
- une unité d'avancement (10) adaptée pour faire avancer les fermetures de récipient (5) le long du trajet d'avancement (P) ;
l'unité d'avancement (10) comprenant :
- un chemin sans fin (24) ;
- une pluralité d'ensembles chariots (25') accouplés mobiles au chemin sans fin (24) et configurés pour interagir avec une ou plusieurs fermetures de récipient (5) accouplées au chemin de guidage (9) ;
- un ensemble d'actionnement configuré pour commander l'avancement des ensembles chariots (25') le long du chemin sans fin (24) pour faire avancer les fermetures de récipient (5) le long du trajet d'avancement (P) ;
chaque ensemble chariot (25') comprenant un chariot (26) accouplé de manière mobile au chemin sans fin (24) et au moins un élément de mise en prise (27) accouplé au chariot respectif (26) et configuré pour venir en prise avec une ou plusieurs fermetures de récipient (5) pour faire avancer la ou les fermetures de récipient (5) le long du trajet d'avancement (P) ;
l'appareil comprenant une chambre d'isolement (8) logeant au moins une partie du chemin de guidage (9) et comprenant au moins une zone de stérilisation (37) à l'intérieur de laquelle les fermetures de récipient (5) avançant le long d'une partie de stérilisation (P1) du trajet d'avancement (P) sont, en cours d'utilisation, stérilisées ; et
- l'appareil comprenant une unité de stérilisation configurée pour stériliser les fermetures de récipient (5) pendant l'avancement des fermetures de récipient (5) le long de la partie de stérilisation (P1) ;
l'élément de mise en prise respectif (27) de chaque ensemble chariot (25') étant agencé à l'intérieur de la chambre d'isolement (8) au moins pendant l'avancement des fermetures de récipient (5) respectives à l'intérieur de la zone de stérilisation (37) ;
le chemin de guidage (9) étant configuré pour supporter et/ou guider les fermetures de récipient (5) pendant l'avancement le long du trajet d'avancement (P) ;
**caractérisé en ce que** :
le chemin sans fin (24) est agencé à l'extérieur de la chambre d'isolement (8) ;
l'ensemble d'actionnement est configuré pour commander chaque ensemble chariot (25') indépendamment des autres ;
chaque ensemble chariot (25') comprenant :
- un premier élément d'interaction (53) accouplé au chariot respectif (26) ; et
- un deuxième élément d'interaction (54) accouplé à l'élément de mise en prise respectif ;
chaque premier élément d'interaction (53) et le deuxième élément d'interaction (54) respectif étant configurés pour interagir l'un avec l'autre au moyen d'une interaction magnétique et/ou électromagnétique de manière à transférer un mouvement du chariot respectif (26) à l'élément de mise en prise respectif (27) ;
chaque élément de mise en prise (27) étant configuré pour venir en prise avec un groupe (29) de fermetures de récipient (5) agencées successivement sur le chemin de guidage (9) ;
chaque élément de mise en prise (27) étant configuré pour entrer en contact avec au moins la fermeture de récipient (5) du groupe (29) de fermetures de récipient (5), qui est agencée en amont des autres le long du trajet d'avancement (P), de manière à appliquer une force de poussée sur le groupe (29) de fermetures de récipient (5) lors de l'avancement du chariot respectif (26) pour faire avancer le groupe (29) de fermetures de récipient (5) le long du trajet d'avancement (P).

2. Appareil de stérilisation selon l'une quelconque des revendications précédentes, l'ensemble d'actionnement comprenant une pluralité de bobines pour former sélectivement un champ électromagnétique et qui sont agencées le long du chemin sans fin (24) ;
chaque ensemble chariot (25') comprenant au moins un élément d'interaction configuré pour interagir avec le champ électromagnétique.

3. Appareil de stérilisation selon l'une quelconque des revendications précédentes, le dispositif de transport comprenant un chemin de stationnement relié au chemin sans fin (24) et adapté pour stocker un ou plusieurs ensembles chariots (25') ;
le chemin de stationnement étant configuré pour permettre l'ajout ou le retrait d'ensembles chariots (25') du chemin sans fin (24), notamment en fonction des conditions de traitement d'une machine de traitement (1 ; 4 ; 6') à l'intérieur de laquelle le dispositif de transport (7') est intégré et/ou à laquelle le dispositif de transport (7') est relié et/ou à laquelle le dispositif de transport (7') achemine, en cours d'utilisation, les fermetures de récipient (5).

4. Appareil de stérilisation selon l'une quelconque des revendications précédentes, le nombre et/ou les vitesses d'avancement des ensembles chariots (25') avançant, en cours d'utilisation, le long du chemin sans fin (24) étant fonction des conditions de traitement d'une machine de traitement (1 ; 4 ; 6') à l'intérieur de laquelle le dispositif de transport (7') est intégré et/ou à laquelle le dispositif de transport (7') est relié et/ou à laquelle le dispositif de transport (7') achemine, en cours d'utilisation, les fermetures de récipient (5).
